# EUROPEAN PATENT APPLICATION

(11) **EP 0 771 564 A1**
(43) Date of publication of application: **07.05.1997**
(21) Application number: 95928004.1
(22) Date of filing: 10.08.1995
(51) Int. Cl.: A61K 31/70, C07H 17/08

(54) **INTERLEUKIN-5 PRODUCTION INHIBITOR**

(30) Priority: 12.08.1994 JP 190288/94; 12.08.1994 JP 190290/94
(71) Applicant: TAISHO PHARMACEUTICAL CO. LTD, Tokyo 171 (JP)
(72) Inventor: HOSHINO, Akihiko Taisho Pharmaceutical Co., Ltd., Tokyo 171 (JP); KASHIMURA, Masato Taisho Pharmaceutical Co., Ltd., Tokyo 171 (JP); ASAKA, Toshifumi Taisho Pharmaceutical Co., Ltd., Tokyo 171 (JP); INOUE, Tomoyuki Taisho Pharmaceutical Co., Ltd., Tokyo 171 (JP); OKUDAIRA, Hirokazu, Tokyo 112 (JP)
(74) Representative: Ruffles, Graham Keith
(86) International application number: JP9501591
(87) International publication number: WO9604919

(57) **Abstract**

Object: to provide an erythromycin derivative having a potent interleukin-5 production inhibitory effect. Constitution: an interleukin-5 production inhibitor containing as the active ingredient an erythromycin derivative, such as 3-0-(4-biphenyl)acetyl-5-0-desosaminyl-6-0-methylerythronolide A 11,12-cyclic carbonate or 3-0-(2-nitro-3,4,5,6-tetrafluoro)phenyl-5-0-desosaminyl-6-0-methylerythronolide A, or a medicinally acceptable acid-addition salt thereof.

## Description

### Technical Field

This invention relates to an inhibitor on interleukin 5 production which contains an erythromycin derivative as an active ingredient.

### Background Art

Interleukin 5 (hereinafter abbreviated as IL-5) is known to be an important factor stimulating differentiation and growth of eosinophils which accelerate allergic inflammation. Therefore, an inhibitor on IL-5 production is useful for the treatment of various allergic diseases, such as bronchial asthma, allergic rhinitis, atopic dermatitis, drug allergy, and eosinophilic pneumonia.

Erythromycin is an antibiotic that has been of wide clinical use as a treating agent for infections caused by Gram-positive bacteria, some kinds of Gram-negative bacteria, Mycoplasma, etc. It has recently been reported that erythromycin and roxithromycin have an inhibitory activity on IL-5 production (Japanese Journal of Allergology, 44(3-2), p. 424 (1993)), but the IL-5 production inhibitory activity of erythromycin and roxithromycin is not sufficient.

An object of this invention is to provide an erythromycin derivative having a potent IL-5 production inhibitory activity.

### Disclosure of Invention

The inventors of the present invention have extensively studied the IL-5 inhibitory activity of erythromycin derivatives, and as a result, have found that the following erythrimycin derivatives exhibit a potent IL-5 production inhibitory activity and thus completed the present invention.

The present invention provides an IL-5 production inhibitor comprising 3-O-(4-biphenyl)acetyl-5-O-desosaminyl-6-O-methylerythronolide A 11,12-cyclic carbonate, 3-O-(2-nitro-3,4,5,6-tetrafluoro)phenyl-5-O-desosaminyl-6-O-methylerythronolide A, or a pharmaceutically acceptable acid addition salt thereof as an active ingredient.

The pharmaceutically acceptable acid addition salt for use in the invention includes an acetate, a propionate, a butyrate, a formate, a trifluoroacetate, a maleate, a tartrate, a citrate, a stearate, a succinate, an ethylsuccinate, a lactobionate, a gluconate, a glucoheptonate, a benzoate, a methanesulfonate, an ethanesulfonate, a 2-hydroxyethanesulfonate, a benzenesulfonate, a p-toluenesulfonate, a laurylsulfate, a malate, an aspartate, a glutamate, an adipate, a cysteinate, a hydrochloride, a hydrobromide, a phosphate, a sulfate, a hydroiodide, a nicotinate, an oxalate, a picrate, a thiocyanate, an undecanoate, a salt of an acrylic acid polymer, and a salt of a carboxyvinyl polymer.

The 3-O-(4-biphenyl)acetyl-5-O-desosaminyl-6-O-methylerythronolide A 11,12-cyclic carbonate used in the invention can be prepared according to, for example, the process described in WO 93/13116.

The 3-O-(2-nitro-3,4,5,6-tetrafluoro)phenyl-5-O-desosaminyl-6-O-methylerythronolide A used in the invention can be prepared by, for example, as follows. Step (1): 5-O-Desosaminyl-6-O-methylerythronolide A dissolved in an appropriate solvent is reacted with pentafluoronitrobenzene in the presence of a base. Suitable solvents include acetone, tetrahydrofuran, N,N-dimethylformamide, dimethyl sulfoxide, dioxane, and mixtures thereof. Suitable bases include sodium hydride, sodium hydroxide, and potassium hydroxide. The reaction temperature ranges from -20° to 50°C, preferably 0° to 25°C.

The compounds according to the invention can be administered orally or non-orally in the form of tablets, capsules, granules, dusts, powders, troaches, ointments, creams, emulsions, suspensions, suppositories, and injectable solutions. These dose forms can be prepared in a conventional manner, for example, the methods specified in Japanese Pharmacopoeia (12th Rev.). An appropriate dose form is chosen depending on the conditions and age of a patient and the purpose of treatment. In the preparation of various dose forms, commonly employed vehicles (e.g., crystallien cellulose, starch, lactose, mannitol), binders (e.g., hydroxypropyl cellulose, polyvinylpyrrolidone), lubricants (e.g., magnesium stearate, talc), disintegrants (e.g., craboxymethyl cellulose calcium), and the like can be used.

The dosage of the compound of the present invention ranges, for example, from 50 to 2000 mg in 2 to 3-divided doses per day in orall administration to adults, while appropriately varying depending on the age, body weight and conditions of a patient.

### Industrial Applicability

The compounds according to the invention exhibit a potent IL-5 production inhibitory activity and are useful as an IL-5 production inhibitor in humans and animals (inclusive of livestock). Thus, the compounds of the invention are effective on diseases caused by IL-5 production, i.e., various allgergic diseases, such as bronchial asthma, allergic rhinitis, atopic dermatitis, drug allergy, and eosinophilic pneumonia.

### Best Mode for Carrying out Invention

The present invention is now illustrated in greater detail with reference to Examples.

### Example 1: Preparation of 3-O-(4-Biphenyl)acetyl-5-O-desosaminyl-6-O-methylerythronolide A 11,12-Cyclic Carbonate

(1) In 100 ml of acetone was dissolved 11.78 g (0.02 mol) of 5-O-desosaminyl-6-O-methylerythronolide A, and 2.27 ml (0.024 mol) of acetic anhydride was added to the solution under cooling with ice, followed by stirring at room temperature for 6 hours. Acetone was removed by evaporation under reduced pressure, and the residue was extracted with dichloromethane. The dichloromethane layer was washed successively with a saturated sodium hydrogencarbonate aqueous solution and a saturated sodium chloride aqueous solution, dried over anhydrous magnesium sulfate, and evaporated under reduced pressure to remove the solvent. The residue was recrystallized from ether/n-hexane to gave 12.17 g of 2'-O-acetyl-5-O-desosaminyl-6-O-methylerythronolide A as white powder.
   Melting point: 158-160°C
   Mass (FAB) m/z: 632 [MH]⁺
   ¹H-NMR (200 MHz, CDCl₃) δ ppm:
   2.07 (3H, s), 2.26 (6H, s), 2.95 (3H, s), 3.26 (1H, s), 3.96 (1H, s)
   IR (KBr), cm⁻¹: 3469, 1750, 1733, 1693
(2) In 500 ml of dichloromethane was dissolved 50 g (84.8 mmol) of the compound obtained in (1) above, and 102.6 ml (1.27 mol) of pyridine was added to the solution under ice-cooling. A solution of 25.4 ml (212 mmol) of trichloromethyl chloroformate in 40 ml of dichloromethane was added thereto dropwise at the same temperature, followed by stirring for 5.5 hours. Cold water and a saturated sodium hydrogencarbonate aqueous solution were added to the reaction mixture in small portions, and the mixture was extracted with dichloromethane. The dichloromethane layer was washed successively with a saturated sodium hydrogencarbonate aqueous solution and a saturated sodium chloride aqueous solution, and dried over anhydrous magnesium sulfate. The solvent was removed by evaporation under reduced pressure. The residue was purified by silica gel column chromatography (eluent: acetone/n-hexane/triethylamine=6 to 10:10:0.2) to give 41.93 g of 2'-O-acetyl-5-O-desosaminyl-6-O-methylerythronolide A 11,12-cyclic carbonate as a white foamy substance.
   ¹H-NMR (200 MHz, CDCl₃) δ ppm:
   2.05 (3H, s), 2.25 (6H, s), 2.92 (3H, s), 4.57 (1H, d, J=9Hz), 4.74 (1H, s), 4.75 (1H, dd, J=10Hz, 9Hz), 5.13 (1H, dd, J=12Hz, 2Hz)
(3) In 30 ml of dichloromethane was dissolved 1.274 g (6 mmol) of 4-biphenylacetic acid, and 0.84 ml (6 mmol) of triethylamine was added thereto. To the solution was added 0.75 ml (6 mmol) of pivaloyl chloride under cooling with ice, followed by stirring for 30 minutes. A solution of 1.65 ml (20.4 mmol) of pyridine and 1.31 g (2 mmol) of the compound obtained in (2) above in 10 ml of dichloromethane was added thereto. After stirring for 18 hours, the reaction mixture was post-treated in the same manner as in (1) above. The solvent was evaporated under reduced pressure, and the resultant crude product was purified by silica gel column chromatography (eluent: acetone/n-hexane/triethylamine =4:10:0.05) to give 580 mg of a pale yellow foamy substance. The resulting compound (580 mg, 0.72 mmol) was heated under reflux in 10 ml of methanol for 3 hours, and the solvent was removed by evaporation under reduced pressure to give 490 mg of the title compound as a pale yellow powder.
   ¹H-NMR (200 MHz, CDCl₃) δ ppm:
   2.21 (6H, s), 3.01 (3H, s), 3.90 (1H, d, J=7Hz), 4.76 (1H, s), 5.10 (1H, d, J=11Hz), 7.30-7.49 (5H, m), 7.55-7.61 (4H, m)

### Example 2: Preparation of 3-O-(2-Nitro-3,4,5,6-tetrafluoro)-phenyl-5-O-desosaminyl-6-O-methylerythronolide A

To a solution of 1.178 g (2 mmol) of 5-O-desosaminyl-6-O-methylerythronolide A and 4.02 ml (10 mmol) of pentafluoronitrobenzene in 10 ml of tetrahydrofuran was added 240 mg (6 mmol) of 60% sodium hydride, followed by stirring at room temperature for 2 hours. The reaction mixture was extracted with ethyl acetate, and the extract was washed with a saturated sodium chloride aqueous solution and dried over anhyrous magnesium sulfate. The solvent was evaporated under reduced pressure, and the residue was purified by silica gel column chromatography (eluent: chloroform/methanol/aqueous ammonia=19:1:0.1) to give 210 mg of the title compound.
Mass (FAB) m/z: 783 [MH]⁺
¹H-NMR (200 MHz, CDCl₃) δ ppm: 2.25 (6H, s), 3.05 (3H, s)

### Example 3:

Ten grams of the compound prepared in Example 1, 550 g of lactose, 300 g of corn starch, 100 g of carboxymethyl cellulose calcium, and 30 g of polyvinylpyrrolidone were mixed well, granulated using ethanol, dried and classified in an usual manner. The granules were mixed with 10 g of magnesium stearate, and the mixture was tableted in a usual manner to obtain tablets each weighing 100 mg.

### Example 4:

The action on IL-5 production in mice was examined according to the following method as disclosed in M. Hikida, et al., Immunology Letters, Vol. 34, pp. 297-302 (1992).

Murine Th2 clone (D10.G4.1 cells) was purchased from ATCC. Antigen presenting cells were prepared by suspending 1 x 10⁷ spleen cells of a 8-week-old female C3H/HeN mice in 5 ml of an RPMI-1640 medium and incubated together with 50 µg/ml of mitomycin C (MMC) at 37°C for 30 minutes and then washed with three 50 ml portions of RPMI-1640. To RPMI-1640 containing 10% bovine serium were added 0.5 U of IL-2 (produced by Genzyme) and 5 x 10⁻⁵ M of 2-mercaptoethanol to prepare a medium for tissue culture. A test compound was dissolved in dimethyl sulfoxide (DMSO) and diluted with the tissue culture medium to have a final DMSO concentration of 0.1% and a varied test compound concentration.

In each well of a 96-well microtiter plate (produced by Corning Glass Works) were put each 50 µl/well of 4 x 10⁵ cells/ml of D10.G4.1 cells, 2 x 10⁶ cells/ml of MMC-treated antigen presenting cells, 400 µg/ml of conalbumin (produced by Sigma) as an antigen and the solution of the test compound in the tissue culture medium (to make 200 µl/well), and incubated in an incubator under 5% CO₂ at 37°C for 48 hours. After completion of the incubation, the supernatant liquor of the culture was collected, and the cells were seprated by centrifugation. The IL-5 in the supernatant liquor was determined with an IL-5 ELISA kit produced by ENDOGEN. The inhibitory effect of the test compound on IL-5 production, expressed in terms of 50% inhibitory concentration (IC₅₀), is shown in Table 1 below.

**TABLE 1**

| Test Compound | IC₅₀ (M) |
|---|---|
| Compound of Example 1 | <4 x 10⁻⁷ |
| Compound of Example 2 | <1 x 10⁻⁷ |
| Roxithromycin | 9.3 x 10⁻⁷ |
| Erythromycin | 4.5 x 10⁻⁶ |

### Example 5:

The compounds of Examples 1 and 2 were orally administered to ICR male mice grouped in fives. No death was observed at a dose level of 100 mg/kg, proving that the compounds are of high safety.

## Claims

1. An interleukin 5 production inhibitor comprising 3-O-(4-biphenyl)acetyl-5-O-desosaminyl-6-O-methylerythronolide A 11,12-cyclic carbonate, 3-O-(2-nitro-3,4,5,6-tetrafluoro)phenyl-5-O-desosaminyl-6-O-methylerythronolide A, or a pharmaceutically acceptable acid addition salt thereof as an active ingredient.

2. Use of 3-O-(4-biphenyl)acetyl-5-O-desosaminyl-6-O-methylerythronolide A 11,12-cyclic carbonate, 3-O-(2-nitro-3,4,5,6-tetrafluoro)phenyl-5-O-desosaminyl-6-O-methylerythronolide A, or a pharmaceutically acceptable acid addition salt thereof for the production of an interleukin 5 production inhibitor.

3. A method for treating an allergic disease comprising administering 3-O-(4-biphenyl)acetyl-5-O-desosaminyl-6-O-methylerythronolide A 11,12-cyclic carbonate, 3-O-(2-nitro-3,4,5,6-tetrafluoro)phenyl-5-O-desosaminyl-6-O-methylerythronolide A, or a pharmaceutically acceptable acid addition salt thereof.
